# EUROPÄISCHE PATENTSCHRIFT

(11) **EP 0 562 258 B1**
(45) Veröffentlichungstag und Bekanntmachung des Hinweises auf die Patenterteilung: **08.05.1996**
(21) Anmeldenummer: 93102289.1
(22) Anmeldetag: 13.02.1993
(51) Int. Cl.: C07C 2/34, B01J 31/26

(54) **Verfahren zur Herstellung von Propenoligomeren**
Method for the production of propene oligomers
Procédé pour l'oligomérisation de propène

(30) Priorität: 27.02.1992 DE 4205932
(43) Veröffentlichungstag der Anmeldung: 29.09.1993
(73) Patentinhaber: BASF Aktiengesellschaft, D-67063 Ludwigshafen (DE)
(72) Erfinder: Mueller, Hans-Joachim, Dr., W-6718 Gruenstadt (DE); Marczinke, Bernd Lothar, Dr., W-6720 Speyer (DE); Kerth, Juergen, Dr., W-6719 Carlsberg (DE); Konrad, Rainer, Dr., W-6701 Goennheim (DE); Schweier, Guenther, Dr., W-6701 Friedelsheim (DE); Strohmeyer, Max, Dr., W-6703 Limburgerhof (DE); Rieger, Bernhard, Dr., W-7401 Nehren (DE)

(56) Entgegenhaltungen:
- EP-A- 0 257 696
- EP-A- 0 268 214
- EP-A- 0 283 739
- EP-A- 0 481 480
- US-A- 5 087 788

## Beschreibung

Die vorliegende Erfindung betrifft ein Verfahren zur Übergangsmetallkomplex-katalysierten Oligomerisierung von Propen in flüssiger Phase zu Propenoligomeren mit einem hohen Gehalt an endständigen Doppelbindungen.

Mit Hilfe von Komplexen der Übergangsmetalle der vierten Nebengruppe des Periodensystems, beispielsweise den Dicyclopentadienylkomplexen des vierwertigen Titans, Zirkoniums oder Hafniums, gelingt es in Gegenwart eines aluminiumorganischen Cokatalysators, wie Methylaluminoxan, Olefine wie Ethen, Propen oder andere 1-Olefine zu hochmolekularen Verbindungen zu polymerisieren (vgl. DE-A 26 08 933).

Oligomere 1-Olefine, insbesondere Oligomere des Propens lassen sich katalytisch auf verschiedenerlei Art herstellen.

Mit Hilfe von π-Allylnickelhalogeniden in Gegenwart von Phosphanen und aluminiumorganischen Verbindungen kann Propen katalytisch dimerisiert werden (Brennstoff-Chemie 49, 323 (1968)). Dieses Verfahren eignet sich gut zur Herstellung von Propen-Dimeren, liefert aber nur sehr geringe Mengen der tri- und tetrameren Oligomeren und ist daher zu deren Herstellung nicht brauchbar.

In Erdöl und Kohle 12, 547 (1959) wird ein Verfahren zur Oligomerisierung von Propen beschrieben, bei dem Aluminiumchlorid in Nitromethan als Katalysator benutzt wird. Der überwiegende Teil der sich bei diesem Verfahren bildenden Propen-Oligomere hat mehr als 24 Kohlenstoffatome, d.h. Propen-Tri- oder Tetramere können nach diesem Verfahren ebenfalls nur in geringer Ausbeute erhalten werden. Darüber hinaus wird das verwendete Katalysatorsystem bereits nach 7 bis 8 Stunden inaktiv und neigt zur explosionsartigen Selbstzersetzung. Somit scheidet dieses Verfahren ebenfalls für eine technische Anwendung aus.

Mit (R)-Ethylen-bis-(4,5,6,7-tetrahydro-1-indenyl)dimethyl-zirkonium und Methylaluminoxan als Cokatalysator werden nach J. Am. Chem. Soc. 109, 6189 (1987) aus Propen in Gegenwart von Wasserstoff gesättigte, oligomere Alkane erhalten.

In Abwesenheit von Wasserstoff können nach Angew. Chem. 101, 1304 (1989) mit einem ähnlichen Katalysatorsystem, nämlich mit (S)-[1,1'-Ethylen-bis-(4,5,6,7-tetrahydro-1-indenyl)]-zirkonium-bis(O-acetyl-(R)-mandelat) (56,6 mg ≙ 0,076 mmol)/Methylaluminoxan (520 mg ≙ 9 mmol Al) gelöst in 155 ml Toluol aus 20,4 g Propen (0,48 mol) Propenoligomere in 88 % Ausbeute erhalten werden. Um dieses Ergebnis zu erzielen ist es allerdings erforderlich, ein großes Katalysator/Alken-Verhältnis einzustellen, d.h. das Propen muß sehr langsam zum Katalysator dosiert werden - im genannten Fall während einer Zeitdauer von 19 Stunden - da ansonsten hauptsächlich Polymere entstehen.

EP-A 268 214 beschreibt ein Verfahren zur Herstellung von Propenoligomeren in An- oder Abwesenheit von Wasserstoff mit Hilfe von aus peralkylierten Cyclopentadienyl-Liganden hergestellten Titanocen-, Zirkonocen- und Hafnocen-Katalysatoren und Aluminoxan-Cokatalysatoren. Derartige peralkylierte Metallocene sind aber ebenso wie die oben beschriebenen Zirkoniumkatalysatoren nur durch aufwendige Synthesen zugänglich und ihre Anwendung in einem großtechnischen Verfahren wegen ihres hohen Preises nicht wirtschaftlich. Titanocene, Zirkonocene und Hafnocene mit unsubstituierten Cyclopentadienyl-Liganden ergeben nach der Lehre dieser Schrift aber keine Oligomeren, sondern führen ausschließlich zur Bildung von Polymeren.

EP-A 257 696 offenbart ein Verfahren zur Dimerisierung von α-Olefinen, wie Propen, mit Hilfe von Zirkonocen- und Hafnocen-Katalysatoren in Gegenwart von Aluminoxan-Cokatalysatoren, wobei ein Atomverhältnis von Aluminium zu Zirkonium bzw. Hafnium von 1 bis 100 gewählt wird. Ein höheres Atomverhältnis von Aluminium zu Zirkonium bzw. Hafnium führt auf Kosten der Dimerbildung zu einer Zunahme der Propenoligomeren im Produkt. Die Produktivität der Katalysatoren zur Herstellung von Propenoligomeren ist bei diesem Verfahren mit 30 bis 700 ml Produkt/g Katalysator x h so gering, daß eine wirtschaftliche Herstellung von Propenoligomeren nach diesem Verfahren nicht möglich ist.

Der vorliegenden Erfindung lag daher die Aufgabe zugrunde, ein Verfahren zur selektiven Herstellung von Propenoligomeren mit einem hohen Gehalt an endständigen Doppelbindungen, insbesondere ein Verfahren zur selektiven Herstellung von ungesättigten Propenoligomeren mit einem hohen Gehalt an Di-, Tri-, Tetra- und Pentameren zu finden, das sich mit Hilfe eines auf einfache und kostengünstige Weise herstellbaren Katalysators wirtschaftlich durchführen läßt. Dabei sollte sich das verwendete Katalysatorsystem außer durch eine hohe Selektivität, insbesondere durch eine hohe Produktivität auszeichnen.

Dementsprechend wurde ein Verfahren zur Übergangsmetallkomplex-katalysierten Oligomerisierung von Propen in flüssiger Phase zu Propenoligomeren mit einem hohen Gehalt an endständigen Doppenbindungen gefunden, das dadurch gekennzeichnet ist, daß man Propen in Gegenwart eines Katalysators der Formel I

Cp₂MX₂ I,

in der Cp eine unsubstituierte Cyclopentadienyl-Einheit und/oder eine Mono-C₁- bis C₄-alkylcyclopentadienyl-Einheit, M ein Zirkonium- oder Hafniumatom ist und in der die Liganden X für ein Hydridanion, ein Halogenanion und/oder eine Methylgruppe stehen und in Gegenwart eines Aluminoxan-Cokatalysators oligomerisiert, dabei den Katalysator I bezüglich des Aluminoxan-Cokatalysators in einem Mengenverhältnis einsetzt, das einem M/Al-Atomverhältnis von 1:250 bis 1:1000 entspricht und Temperaturen von 50 bis 110°C und einen Druck von 30 bis 100 bar anwendet.

Bei den Katalysatoren I handelt es sich um sogenannte Zirkonocene und Hafnocene, mithin um Komplexe des vierwertigen Zirkoniums und Hafniums, bei denen das Metallatom M sandwichartig zwischen zwei unsubstituierte und/oder C₁- bis C₄-Monoalkyl-substituierte Cyclopentadienyl-Gruppen Cp gebunden ist, wobei die restlichen Valenzen des Zentralatoms M durch Hydrid- und/oder Halogenanionen und/oder durch Methylgruppen abgesättigt sind. Besonders bevorzugt werden solche Zirkonocen- und Hafnocenkatalysatoren im erfindungsgemäßen Verfahren verwendet, deren Cyclopentadienyl-Gruppen unsubstituiert sind. Als Halogenanionen können sowohl Fluor-, Chlor-, Brom- und/oder Jodanionen an das Metallatom gebunden sein.

Beispiele für geeignete Katalysatoren sind:
Cp₂ZrF₂, Cp₂ZrCl₂, Cp₂ZrBr₂, Cp₂ZrJ₂, Cp₂ZrHCl, Cp₂Zr(CH₃)Cl, Cp₂Zr(CH₃)₂, Cp₂HfF₂, Cp₂HfCl₂, Cp₂HfBr₂, Cp₂HfJ₂, Cp₂HfHCl, Cp₂Hf(CH₃)Cl, Cp₂Hf(CH₃)₂.

Zweckmäßigerweise wird bei der Oligomerisierung nur ein Katalysator eingesetzt, es ist aber auch möglich, Mischungen verschiedener Katalysatoren zu verwenden. Bevorzugte Liganden X sind im erfindungsgemäßen Verfahren Chlorid, Hydrid und die Methylgruppe, als Zentralatom M wird für die Katalysatoren I im erfindungsgemäßen Verfahren Zirkonium besonders bevorzugt. Besonders bevorzugt wird im erfindungsgemäßen Verfahren Zirkonocendichlorid der Formel Ia

Cp₂ZrCl₂ Ia

als Katalysator I benutzt, dessen Cyclopentadienylgruppen unsubstituiert sind.

Die Katalysatoren I können auf einfache Weise nach bekannten Verfahren, z.B. nach Brauer (Hrsg.): Handbuch der Präparativen, Anorganischen Chemie, Band 2, 3. Auflage, Seite 1395 bis 1397, Enke, Stuttgart 1978 synthetisiert werden.

Als Cokatalysatoren werden aluminiumorganische Verbindungen, vorzugsweise Aluminoxane verwendet. Aluminoxane bilden sich bei der partiellen Hydrolyse aluminiumorganischer Verbindungen, beispielsweise solcher der allgemeinen Formeln AlR₃, AlR₂Y und Al₂R₃Y₃, in denen die Reste R z.B. für C₁- bis C₁₀-Alkylgruppen, vorzugsweise C₁- bis C₅-Alkylgruppen für C₃- bis C₁₀-Cycloalkylgruppen, C₇- bis C₁₂-Aralkyl- oder Alkarylgruppen und/oder eine Phenyl- oder Naphthylgruppe stehen können und in denen Y ein Wasserstoffatom, ein Halogenatom, vorzugsweise ein Chlor- oder Bromatom oder eine C₁- bis C₁₀-Alkoxygruppe, vorzugsweise eine Methoxy- oder Ethoxygruppe, sein kann. Die partielle Hydrolyse derartiger aluminiumorganischer Verbindungen kann nach verschiedenerlei Verfahren, z.B. nach dem Verfahren der DE-A 3 240 383 oder nach dem in EP-A 268 214 angegebenen, erfolgen. Die dabei entstehenden, sauerstoffhaltigen Aluminoxane sind im allgemeinen keine einheitlichen Verbindungen, sondern Oligomerengemische der allgemeinen Formel II 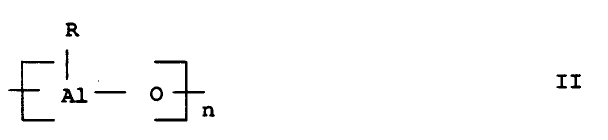
in der n in der Regel eine Zahl von 6 bis 20 ist und R die oben genannte Bedeutung hat. Werden aluminiumorganische Verbindungen mit verschiedenen Resten R oder Gemische aluminiumorganischer Verbindungen mit unterschiedlichen Resten R hydrolysiert, so entstehen Aluminoxane II mit verschiedenerlei Resten R, die ebenfalls als Cokatalysator im erfindungsgemäßen Verfahren eingesetzt werden können. Zweckmäßigerweise werden allerdings Aluminoxane als Cokatalysatoren verwendet, in denen die Reste R gleich sind. Es können auch Mischungen verschiedener Aluminoxane als Cokatalysatoren benutzt werden. Als bevorzugtes Aluminoxan dient im erfindungsgemäßen Verfahren Methylaluminoxan. Da die erfindungsgemäß als Cokatalysatoren verwendeten Aluminoxane, bedingt durch ihre Herstellungsweise, keine einheitlichen Verbindungen sind, wird im folgenden die Molarität von Aluminoxanlösungen auf deren Aluminiumgehalt bezogen.

Zur Oligomerisierung wird der Katalysator I bezüglich des Cokatalysators in einer Menge eingesetzt, die einem M/Al-Atomverhältnis von im allgemeinen 1:250 bis 1:1000, vorzugsweise von 1:300 bis 1:600 und besonders bevorzugt von 1:400 bis 1:500 entspricht.

Die Oligomerisierung des Propens wird vorteilhaft in flüssiger Phase und in einem Lösungsmittel, zweckmäßigerweise unter Verwendung geringer Mengen eines Lösungsmittels, vorzugsweise einem aliphatischen oder aromatischen Kohlenwasserstoff, wie Benzol, Toluol, Xylol, Ethylbenzol, Cumol, Naphthalin, Tetralin, Hexan, Heptan, Octan, Isooctan, Nonan, Decan, Dodecan, Cyclohexan, Decalin, Petrolether oder Ligroin vorgenommen. Als besonders bevorzugte Lösungsmittel werden Toluol und Xylol verwendet. Im erfindungsgemäßen Verfahren werden Lösungsmittel/Propen-Volumenverhältnisse von im allgemeinen 1:20 bis 1:500, vorzugsweise von 1:30 bis 1:200 und besonders bevorzugt von 1:40 bis 1:100 eingestellt, wobei sich das Volumen des Propens auf dessen Volumen beim jeweils angewandten Druck bezieht. Unter den angewandten Bedingungen ist das Propen flüssig.

Die erfindungsgemäße Oligomerisierung wird im allgemeinen bei Temperaturen von 50 bis 110°C, besonders bevorzugt bei 60 bis 90°C und bei einem Druck von 30 bis 100, vorzugsweise von 30 bis 50 bar ausgeführt. Das Metallocen/Propen-Verhältnis ist in der Regel nicht kritisch für das erfindungsgemäße Verfahren, zweckmäßigerweise werden allerdings Metallocen/Propen-Molverhältnisse von 1:50 bis 1:250000, vorzugsweise von 1:70 bis 1:200000 und insbesondere von 1:90 bis 1:190000 angewandt.

Das erfindungsgemäße Verfahren kann sowohl chargenweise, z.B. in Rührautoklaven, oder kontinuierlich, beispielsweise in Rohrreaktoren, durchgeführt werden. Nach der Abtrennung des Katalysators durch Destillation der Produkte oder durch dessen Hydrolyse und anschließende Filtration der ausgefallenen Feststoffe wird das Reaktionsgemisch zweckmäßigerweise destillativ, gewünschtenfalls bei vermindertem Druck, aufgearbeitet.

Das im erfindungsgemäßen Verfahren als Rohstoff verwendete Propen kann aus vielerlei Quellen stammen, z.B. von Crack-Gasen, beispielsweise aus Steamcrackern. Ebenso kann Propen verwendet werden, wie es z.B. bei der Propandehydrierung gebildet wird. Der Einsatz von Propen aus anderen Quellen ist ebenfalls möglich. Das Propen kann in gereinigter Form eingesetzt werden, es kann allerdings auch in Gemischen mit anderen Kohlenwasserstoffen, die sich unter den Bedingungen der Umsetzung inert verhalten, eingesetzt werden.

Das erfindungsgemäße Verfahren ermöglicht die selektive Herstellung von Propenoligomeren mit endständigen Doppelbindungen, insbesondere die selektive Herstellung von Propenoligomeren mit einem hohen Anteil an Propen-Tri-, Tetra- und Pentameren und mit hohen Produktivitäten.

### Beispiele

### Beispiel 1

In einem 2 l-Rührautoklaven wurden 30 ml 1,5 molare Methylaluminoxanlösung in Toluol vorgelegt, 900 ml (13,3 Mol) flüssiges Propen aufkondensiert und auf 60°C erwärmt. Dabei stellte sich ein Druck von 20 bar ein. Anschließend wurden 40,5 mg (0,17 mmol) Zirkonocen (Dicyclopentadienyl-zirkoniumdichlorid), gelöst in 7 ml einer 1,5 molaren Methylaluminoxanlösung in Toluol, zugegeben und während einer Zeitdauer von 60 Minuten oligomerisiert. Das Aluminium/Zirkonium-Atomverhältnis betrug 250:1. Es wurde eine Ausbeute von 590 ml Oligomerengemisch erhalten. Die Produktivität des Katalysators unter den angewandten Reaktionsbedingungen, ausgedrückt als ml Produkt/g Katalysator x h, betrug 11900. Die gaschromatographische Analyse des Produkts ergab die folgende Zusammensetzung:

| | |
|---|---|
| Dimere: | 17,5 % |
| Trimere: | 25,9 % |
| Tetramere: | 17,5 % |
| Pentamere: | 6,9 % |
| Hexamere: | 2,5 % |
| Heptamere: | 0,5 % |
| Höhere Oligomere | 29,2 % |

Das Dimere bestand zu 5 % aus 2,3-Dimethylbuten-1 und 91,5 % 2-Methylpenten-1. Das Trimere bestand zu 7 % aus 2,4,5-Trimethylhexen-1 und 87,5 % 2,4-Dimethylhepten-1. Das Tetramere lag in Form zweier näher nicht identifizierter Hauptisomere mit einem Anteil von jeweils 47,0 und 40,0 % vor. Die infrarot- und NMR-spektroskopische Analyse der erhaltenen Produkte belegt, daß ausschließlich Kohlenwasserstoffe mit endständigen Doppelbindungen, die überwiegend in Vinylidengruppen lokalisiert sind, gebildet wurden.

Entsprechend Beispiel 1 wurden die Beispiele 2 bis 4 durchgeführt, wobei die in der Tabelle angegebenen unterschiedlichen Aluminium/Zirkonium-Atomverhältnisse angewandt wurden.

**Tabelle**

| Beispiel | Zirkonocen [mmol] | Methylaluminoxan [mmol] | Al/Zr-Atomverhältnis | Produktivität [ml Produkt/g Kat x h] |
|---|---|---|---|---|
| 2 | 0,17 | 90 | 530 | 14600 |
| 3 | 0,07 | 30 | 430 | 18600 |
| 4 | 0,07 | 60,5 | 860 | 20100 |

## Patentansprüche

1. Verfahren zur Übergangsmetallkomplex-katalysierten Oligomerisierung von Propen in flüssiger Phase zu Propenoligomeren mit einem hohen Gehalt an endständigen Doppelbindungen, dadurch gekennzeichnet, daß man Propen in Gegenwart eines Katalysators der Formel I
Cp₂MX₂ I,
in der Cp eine unsubstituierte Cyclopentadienyl-Einheit und/oder eine Mono-C₁- bis C₄-alkyl-cyclopentadienyl-Einheit, M ein Zirkonium-oder Hafniumatom ist und in der die Liganden X für Hydrid- und/oder Halogenanionen und/oder eine Methylgruppe stehen, und in Gegenwart eines Aluminoxan-Cokatalysators oligomerisiert, dabei den Katalysator I bezüglich des Aluminoxan-Cokatalysators in einem Mengenverhältnis einsetzt, das einem M/Al-Atomverhältnis von 1:250 bis 1:1000 entspricht und Temperaturen von 50 bis 110°C und einen Druck von 30 bis 100 bar anwendet.

2. Verfahren nach Anspruch 1, dadurch gekennzeichnet, daß man als Übergangsmetallkomponente des Katalysators I Zirkonium verwendet.

3. Verfahren nach Anspruch 1, dadurch gekennzeichnet, daß man als Katalysators Zirkonocendichlorid der Formel Ia
Cp₂ZrCl₂ Ia
einsetzt.

4. Verfahren nach Anspruch 1, dadurch gekennzeichnet, daß man als Cokatalysator Methylaluminoxan verwendet.

## Claims

1. A process for propene oligomerization catalyzed by transition metal complexes in liquid phase to give propene oligomers with a high content of terminal double bonds, which comprises oligomerizing propene in the presence of a catalyst of the formula I
Cp₂MX₂ I
where Cp is an unsubstituted cyclopentadienyl unit and/or a mono-C₁-C₄-alkylcyclopentadienyl unit, M is zirconium or hafnium and the ligands X are each hydride and/or halide and/or methyl, and in the presence of an aluminoxane cocatalyst, and where the ratio of the amounts of the catalyst I and the aluminoxane cocatalyst is such that the M/Al atomic ratio is from 1:250 to 1:1000, employing a temperature of from 50 to 110°C and a pressure of from 30 to 100 bar.

2. A process as claimed in claim 1, wherein zirconium is used as transition metal component of the catalyst I.

3. A process as claimed in claim 1, wherein zirconocene dichloride of the formula Ia
Cp₂ZrCl₂ Ia
is employed as catalyst.

4. A process as claimed in claim 1, wherein methylaluminoxane is used as cocatalyst.

## Revendications

1. Procédé d'oligomérisation du propène catalysée par des complexes de métaux de transition en phase liquide, de manière à obtenir des oligomères du propène d'une teneur élevée en doubles liaisons terminales, caractérisé en ce que l'on oligomérise le propène en présence d'un catalyseur de la formule I :
Cp₂MX₂ I
dans laquelle Cp représente une unité cyclopentadiényle non substituée et/ou une unité monoalkyle(C₁ à C₄)cyclopentadiényle, M représente un atome de zirconium ou d'hafnium et les ligandes X représentent chacun un radical hydrure et/ou un anion d'halogène et/ou le radical méthyle, et en présence d'un cocatalyseur d'aluminoxanne, en mettant en oeuvre le catalyseur I par rapport au cocatalyseur d'aluminoxanne dans un rapport quantitatif tel, qu'il corresponde à un rapport atomique M/Al de 1:250 à 1:1000 et on utilise des températures de 50 à 110°C et une pression de 30 à 100 bars.

2. Procédé suivant la revendication 1, caractérisé en ce que l'on utilise le zirconium à titre de composant de métal de transition du catalyseur I.

3. Procédé suivant la revendication 1, caractérisé en ce que l'on utilise, à titre de catalyseur, le dichlorure de zirconocène de la formule Ia :
Cp₂ZrCl₂ Ia

4. Procédé suivant la revendication 1, caractérisé en ce que l'on utilise le méthylaluminoxanne à titre de cocatalyseur.
